# EUROPÄISCHE PATENTSCHRIFT

(11) **EP 3 041 924 B1**
(45) Veröffentlichungstag und Bekanntmachung des Hinweises auf die Patenterteilung: **25.04.2018**
(21) Anmeldenummer: 14789780.5
(22) Anmeldetag: 03.09.2014
(51) Int. Cl.: C12M 1/00

(54) **VORRICHTUNG ZUR GEWINNUNG VON PHYTOPLANKTON (MIKROALGEN)**
DEVICE FOR OBTAINING PHYTOPLANKTON (MICROALGAE)
DISPOSITIF D'EXTRACTION DE PHYTOPLANCTON (MICROALGUES)

(30) Priorität: 06.09.2013 DE 102013109747
(43) Veröffentlichungstag der Anmeldung: 13.07.2016
(73) Patentinhaber: Weber GmbH, 63741 Aschaffenburg (DE)
(72) Erfinder: TÜBBECKE, Gabriele, 63843 Niedernberg (DE); IHLOW, Bernd, 63599 Biebergemünd (DE); WEBER, Reinhard, 63741 Aschaffenburg (DE)
(74) Vertreter: Pöhner, Wilfried Anton
(86) Internationale Anmeldenummer: PCT/DE2014/100319
(87) Internationale Veröffentlichungsnummer: WO 2015/032389

(56) Entgegenhaltungen:
- DE-A1-102008 026 829
- US-A1- 2008 220 515
- US-A1- 2010 323 436
- US-A1- 2012 003 734

## Beschreibung

Die Erfindung bezieht sich auf eine Vorrichtung zur Gewinnung von Phytoplankton (Mikroalgen), bei der in einem Gehäuse eine Nährstofflösung und mehrere vertikal ausgerichtete und in horizontalem Abstand zueinander angeordnete Platten vorhanden sind, die alternierend entweder am Boden oder an der Oberseite des Gehäuses befestigt sind und sich nicht bis zur gegenüberliegenden Wand erstrecken, um eine vertikal mäanderförmige Strömung auszubilden sowie die Nährstofflösung über eine Pumpe umgewälzt wird. Bekannt sind Vorrichtungen, häufig als Photobioreaktoren bezeichnet, mit deren Hilfe Mikroorganismen und hier insbesondere Algen kultiviert und vermehrt werden können. Derartige Photobioreaktoren nutzen zu Wachstum und Vermehrung Kohlenstoffdioxid CO₂ und Sonnenlicht, um Photosynthese zu betreiben. Die verschiedenen Arten und Typen von Mikroorganismen, die mit Hilfe derartiger Bioreaktoren Vermehrung finden können, ist unübersehbar. Ein wirtschaftlich hochinteressanter Bereich des Einsatzes derartiger Vorrichtung ist die Vermehrung von Algen, die sich in aller Regel im Wasser befinden und die eine Vielzahl unterschiedlicher Algenarten von etwa 10.000 an der Zahl umfassen.

DE 10 2008 026829 A1 offenbart eine Beckenanlage mit Trennwände, die von der Oberseite der Beckenanlage ausgehen und nach unten zu sich erstrecken. Dazwischen befinden sich jeweils mittig Seitenwände, die zur Erzeugung und Aufrechterhaltung einer mäanderförmigen Strömung unabdingbar sind. Die wirtschaftliche Bedeutung der Bioreaktoren, insbesondere auch der Algenreaktoren, resultiert daraus, dass eine vielfältige wirtschaftliche, in der Bedeutung immer mehr zunehmende Nutzung möglich ist. So besitzen Algen einen sehr hohen Anteil an Mineralstoffen und Spurenelementen, Vitaminen, ungesättigten Fettsäuren und einen hohen Anteil an Kohlenhydrate, die sie zur Verwendung als Nahrungsmittel oder Nahrungsmittelbestandteile nutzbar erscheinen lassen. Sie werden auch häufig als Zusatzstoffe in Kosmetika oder aufgrund ihres hohen Nährstoffgehaltes in der Medizin und in der Pharmazie als Nahrungsergänzungsmittel eingesetzt, wo sie neben der Beigabe als Komponente auch als Material zur Herstellung von Kapseln Verwendung finden. Die im Zusammenhang mit der vorliegenden Entwicklung schwerpunktmäßig besonders erprobte Süßwasseralge Chlorella Vulgaris enthält z.B. die Mineralstoffe Kalzium, Magnesium, Zink, Eisen, Selen sowie alle essentiellen Aminosäuren und zahlreiche ungesättigte Fettsäuren. Die Nutzung als Nahrungsergänzungsmittel bietet sich aufgrund der vielfältigen gesundheitlichen Vorteile besonders an. Unter Umweltgesichtspunkten ist besonders der CO₂-Verbrauch von Interesse, da durch die Algen die Fixierung von Kohlenstoffdioxid CO₂ möglich ist. Dies erweist sich im Hinblick auf den Emissionshandel als besonders interessanter Aspekt. Eine weitere Einsatzmöglichkeit gilt der Gewinnung von Biotreibstoffen oder von Wasserstoff. Der Vorteil der Kultivierung von Algen im Vergleich zum Anbau von Nutzpflanzen auf dem Land sind der hohe Flächenertrag, das Fehlen von Abfällen, wie z.B. Laub und Wurzeln und der geringe Wasserverbrauch. Zudem stehen sie nicht in Konkurrenz zur Züchtung von Pflanzen, da sie auch dort kultiviert werden können, wo keine Landwirtschaft möglich wäre, sodass sie der Nahrungsmittelproduktion selbst keine Fläche wegnehmen. Aber auch in der Medizin werden bestimmte Algentypen zur Bindung und Ausleitung von Schwermetallen eingesetzt.

Vorliegende Erfindung befasst sich einzig mit jenen im Wasser befindlichen Algen, die das Phytoplankton, den photoautotrophen Teil des Planktons bilden. Der Begriff Mikroalgen wird Synonym zum Begriff Phytoplankton verwendet.

Zur Ernte der Algen muss die Trägerflüssigkeit zentrifugiert werden, da sich die Algen aufgrund ihrer kleinen Größe nicht von alleine absetzen. In Abhängigkeit von dem anschließenden Verwendungszweck ist mitunter eine Trocknung vorzunehmen.

Die Produktion selbst erfolgt im Stand der Technik entweder chargenweise mit Hilfe einer Beuteltechnik oder aber in aufwendig gestalteten Glasröhrensystemen.

Entscheidend ist, dass aufgrund der hohen Kosten resultierend aus dem Fehlen wirtschaftlich nutzbarer großtechnischer Verfahren die Herstellung kostenintensiv ist und die Verwendung von Mikroalgen bislang trotz der bestehenden breiten Anwendungspalette nur auf einen engen Einsatzbereich begrenzt geblieben ist.

Hiervon ausgehend hat sich die Erfindung die Aufgabe gestellt, eine Vorrichtung zur Gewinnung von Phytoplankton zu entwickeln, durch die eine wesentlich wirtschaftlichere Produktion und Gewinnung von Mikroalgen möglich wird.

Gelöst wird diese Aufgabe erfindungsgemäß dadurch, dass
an der im Bereich der Befestigung befindlichen Stirnfläche der Platte Beleuchtungsmittel angebracht sind und die Platte aus transparentem Festmaterial besteht, in das Lichtstreuende Partikel derart eingebettet sind, dass die Dichte des Lichtaustrittes über die Oberfläche der Platte in etwa konstant ist.

Der grundlegende Aufbau der Vorrichtung ist wie folgt:
In einem Gehäuse befindet sich eine Nährstofflösung zur Aufzucht und Vermehrung von Phytoplankton. Während des Betriebes unterliegt die Konzentration der Nährstofflösung aufgrund des stetigen Verbrauches einer gewissen Verminderung, die es notwendig macht, von Zeit zu Zeit die Nährstoffe zuzuführen und zu ergänzen. In diesem Gehäuse befinden sich weiterhin vertikale ausgerichtete und in horizontalem Abstand zueinander angeordnete Platten. Jede der Platten ist entweder am Boden oder an der Oberseite des Gehäuses direkt oder indirekt befestigt und derart dimensioniert, dass sich die Platte nicht bis zur gegenüberliegenden Wandung des Gehäuses erstreckt. Es verbleibt demnach ein gewisser Abstand also eine Lücke, durch den die Nährstofflösung von dem durch zwei benachbarte Platten gebildeten Zwischenraum in den nächsten Zwischenraum, entweder im Bereich der Oberseite oder am Boden des Gehäuses, überströmt. Bei entsprechender Zirkulation der Nährstofflösung hält man im Ergebnis eine vertikal meanderförmige Strömung. Zum Zwecke der Umwälzung ist eine Pumpe vorgesehen, die derart zu gestalten ist, dass das Phytoplankton möglichst wenig beeinträchtigt und beschädigt wird.

In jenem Bereich der Platte, in dem die Befestigung erfolgt, werden an der Stirnfläche Beleuchtungsmittel angebracht. Nachdem die Platte aus transparentem Festmaterial besteht, wird das von den Beleuchtungsmitteln emittierte Licht in die Platte eintreten und sich dort ausbreiten. Es gelten dann die optischen Gesetze der Lichtbrechung, die dazu führen, dass eine Totalreflexion an der Oberfläche der Platte und in deren Inneren stattfindet. Durch das Einbetten lichtstreuender Partikel in das Plattenmaterial wird die Totalreflexion reduziert und ein Lichtaustritt erfolgt, vornehmlich unter kleinem Winkel zur Plattenoberfläche, wodurch sich im Ergebnis eine leuchtende Oberfläche erzeugen lässt. Die eingebetteten Partikel haben die Aufgabe, gezielt und an den jeweiligen Stellen die Totalreflexion punktuell aufzuheben und nach Art eines Spiegels das dort auftreffende und in seinem Querschnitt im Vergleich zur gesamten Fläche des Lichtstroms kleine Lichtbündel zu reflektieren und umzulenken, sodass die Lichtstrahlen dann die Platte an den Oberflächen verlassen können. Durch entsprechende Anordnung der Partikel im Inneren der Platte d.h. durch die Wahl einer geeigneten, ortsabhängigen Partikeldichte in der Platte lässt sich erreichen, dass die Dichte des Lichtaustrittes über die gesamte Oberfläche betrachtet nahezu konstant ist.

In der Nährstofflösung angelangt, entfaltet das Licht seine gewünschte Wirkung, in dem das Phytoplankton in Gegenwart von CO₂ zur Photosynthese angeregt und dadurch die Vermehrung und das Wachstum des Phytoplanktons wesentlich unterstützt wird. Durch die Zufuhr von und Unterstützung mit Licht erhält man eine wesentlich höhere Ausbeute an Phytoplankton (Mikroalgen).

Die durch Verwendung der vorgeschlagenen Vorrichtung und bei Durchführung des entsprechenden Verfahrens erreichbaren Vorteile sind erheblich. Die Erhöhung der Ausbeute kommt nicht nur dadurch zustande, dass durch das großflächige Einbringen von Licht die Photosynthese unterstützt wird, sondern auch dadurch, dass aufgrund der Beleuchtungsmittel ein 24-Stunden-Betrieb möglich wird, der im Vergleich zu den mit Sonnenlicht betriebenen Bioreaktoren ein Vielfaches an Ausbeute zulässt. Als weiteres ist als Vorzug anzusehen, dass der Ort der Befestigung der Beleuchtungsmittel nach außen zu, also im Randbereich ausschließlich stattfindet, sodass eine Reparatur oder ein Austausch der Beleuchtungsmittel aufgrund deren unmittelbaren Zugänglichkeit ohne weiteres möglich ist und insbesondere nicht erfordert, die Platten zu demontieren. Die in vertikaler Richtung erfolgende mäanderförmige Durchströmung des Gehäuses erlaubt eine hohe Ausbeute bei minimalem Eigenbedarf an Grundfläche.

Der Begriff Beleuchtungsmittel ist im Sinne der Erfindung allgemein auszulegen. So können hierunter Leuchtstoffröhren, Glühlampen, Halogenstrahler und LED's und OLED's verstanden werden. Von besonderem Vorteil ist, hierbei LED's einzusetzen, die bei geringem Energieaufwand eine frequenzselektive Emission gestatten.

Für die hier im Vordergrund des Interesses stehenden Süßwasseralgen Chlorella Vulgaris sind die Frequenzen aus dem Spektralbereich 430 +/- 10 nm somit aus dem blauen Farbspektrum und/oder der Spektralbereich aus dem roten Spektrum von 680 +/- 10 nm besonders effizient. LEDs, die aus diesem Frequenzbereich Licht emittieren, führen bei minimalstem Energieaufwand zu größten Wirkungen.

Obwohl es ausreichend ist, bereits einen einzigen der vorgenannten Frequenzbereichen zu applizieren, hat sich als optimal herausgestellt, eine Mischung von 70 (rotes Spektrum) zu 30 (blaues Spektrum) der oben genannten Frequenzspektren einzusetzen. Selbstredend ist, dass auch weißes Licht eingesetzt werden kann, welches eine oder beide der Frequenzbereiche emittiert. Der Nachteil wäre hier, dass der Emissionsquelle Energie zugeführt werden müsste, die zur Emission auch jener Frequenzbereiche Anlass geben würde, die ohne nennenswerten Nutzen sind. Dies ist unter energetischen Gesichtspunkten von Nachteil.

Als beispielhaft für das Plattenmaterial wird Acrylglas genannt, das in der Lage ist, das an der Stirnfläche zugeführte Licht gleichmäßig zu verteilen und auch die Möglichkeit eröffnet, lichtstreuende Partikel einzubringen.

Von besonderem Vorteil ist, auf den dem Lichtaustritt dienenden Oberflächen der Platten durch Satinierung eine zusätzlich Maßnahme vorzusehen, die für eine (weitere) Vergleichmäßigung des Lichtaustrittes Sorge trägt. Unter dem Begriff "Satinierung" wird entsprechend üblicher Terminologie eine Oberflächenbehandlung beschrieben, durch die eine "Rauhigkeit" entsteht, welches zu einer weiteren Vergleichmäßigung und Homogenisierung des Lichtes beiträgt. Nachdem der Lichtaustritt quantitativ über jene Oberflächen austritt, die sich an die Stirnflächen der Platten anschließen (und nicht über die umlaufenden Stirnflächen selbst), empfiehlt sich nur die großen Flächen, also hier die Oberflächen der Platten zu satinieren.

Zur Aufrechterhaltung der mäanderförmigen Strömung ist in der Regel der Einsatz von Pumpen erforderlich. Die Auswahl der geeigneten Pumpentypen aus der Vielzahl der zur Verfügung stehenden Möglichkeiten hat sein Augemerk darauf zu richten, dass die in der Nährstofflösung befindlichen Algen möglichst wenig geschädigt oder gar getötet werden, was sich im Hinblick auf die Leistung der Anlage negativ bemerkbar machen würde. Als besonders geeignet sieht die Erfindung der Verwendung einer Exzenterschneckenpumpe an.

Sobald die Algendichte die gewünschte Konzentration erreicht hat, ist die Nährstofflösung zumindest teilweise auszuleiten und in einer separaten Station die Algen von der Nährstofflösung zu trennen. Zu diesem Zwecke werden in aller Regel Zentrifugen eingesetzt. Als Verfahrensprodukt werden die Mikroalgen, ggf. nach einer Trocknung, in den Verkehr gebracht; die verbleibende Nährstofflösung wird in den Kreislauf zurückgeführt.

Aus diesem Grunde ist besonders angezeigt, die Vorrichtung in geschlossenem Kreislauf zu betreiben, d.h. die am Ende der Vorrichtung vorhandene und mit Algen bestückte Nährstofflösung wieder dem Eingang zuzuführen und diesen Vorgang so lange zu wiederholen, bis die gewünschte Algendichte angereichert wird und die erneute (teilweise) Ausleitung erfolgen kann.

Zur Unterstützung der durch externe Energiezufuhr zu speisenden Beleuchtungsmittel können als zusätzliche Maßnahmen das natürliche Sonnenlicht genutzt werden, indem die Sonnenstrahlung eingefangen und auch auf die Stirnfläche der Platten fokussiert und geleitet wird. Auf diese Weise erfolgt eine Unterstützung der Beleuchtung der Platte aus einer kostenlosen Energiequelle, die es zulässt, dass die Leistung der Beleuchtungsmittel heruntergefahren und somit Energie eingespart werden kann.

Aufgrund der Anordnung der Platten wird die Strömung der Nährstofflösung an der Stirnfläche, die den Beleuchtungsmitteln gegenüber liegt, also beispielsweise im Bodenbereich, herumgeführt. Gleiches findet bei alternierender Anordnung an der Oberseite der nächsten Platte statt, indem die Nährstofflösung an der Oberseite überströmt. Besonders im unteren Bereich der Umlenkung, also dort wo das gesamte Volumen von Nährstofflösung ausgefüllt ist, besteht Anlass Leitbleche in Form von Zylinderhalbschalen vorzusehen, die gewährleisten, dass eine weitgehend turbulenzfreie Strömung in diesen Bereichen erfolgt. Anderenfalls besteht die Gefahr der Ausbildung von Turbulenzen, durch welche der Durchfluss erschwert und behindert und die Ausbildung eines homogenen Algenwachstums geschwächt wäre.

Von Vorteil ist es, im Falle der Existenz von Leitblechen neben den ebenen Platten auch diese Leitbleche entsprechend den Platten aufzubauen, d.h. von deren Stirnflächen her, im Falle von halbzylinderförmigen Schalen, also von der in Richtung der Längsachse verlaufenden Kanten, auch dort mit Hilfe von Beleuchtungsmittel Licht einzubringen. Im Ergebnis erhält man eine intensivere Nutzung und höhere Ausbeute durch die effektive Vergrößerung der der Photosynthese zur Verfügung stehenden Flächen.

Die erfindungsgemäßen Vorrichtungen sind im Hinblick auf die avisierte Leistungskapazität entsprechend zu dimensionieren. Hierzu wird vorgeschlagen, die einzelne Vorrichtung als Modul zu konzipieren, um auf technisch einfache Weise durch ein Hintereinanderschalten oder paralleles Anordnen mehrerer einzelner Module die gewünschte Leistung generieren zu können. Im Falle der Reihenschaltung der einzelnen Module findet der Kreislauf von dem in Strömungsrichtung gesehen letzten Modul zum ersten Modul der Reihe statt, sodass die Nährstofflösung erst dann wieder zum ersten Modul zurückgeführt wird, wenn alle Module der Reihe durchlaufen wurden und eine entsprechende Anreicherung erfolgt ist.
Bei Parallelschaltung von n Modulen erhöht sich die Leistungsfähigkeit um das n-fache.

Eine weitere Maßnahme zur Leistungssteigerung der Anlage sind Messfühler zur Erfassung der Temperatur und/oder elektrischen Leitfähigkeit und/oder dem pH-Wert und/oder der Sauerstoffkonzentration und/oder der optischen Dichte vorhanden. Auf diese Weise lassen sich die Ist-Werte des Betriebszustandes erfassen und die Möglichkeit eröffnet, durch Einflussnahme von außen die Produktionsparameter in einen optimalen Zustand zu fahren. Die Sauerstoffkonzentration ist ein Maß für die stattfindende Photosynthese. Die optische Dichte lässt erkennen, wie hoch die Algendichte in der Nährstofflösung ist und gilt als Indikator dafür, wann die Nährstofflösung mit dem Phytoplankton (Mikroalgen) soweit angereichert ist, dass die Nährstofflösung abgezweigt und von den Phytoplankton getrennt werden muss. Zur Optimierung werden hierzu Maßnahmen ergriffen, wie sie von dem Gebiet der Steuertechnik allgemein bekannt sind und Anwendung finden.

Zur Unterstützung der Photosynthese ist es zweckmäßig, Leitungen zur Einspeisung von CO₂ und/oder Leitungen zum Absaugen von O₂ anzuordnen.

Der Betrieb der Vorrichtung geschieht in der Weise, dass zunächst eine Nährstofflösung, ggf. bereits mit Phytoplankton dotiert, in die Vorrichtung eingebracht wird, die Nährstofflösung an den mit austretendem Licht versorgten Oberflächen der Platten vorbeigeführt wird, so dass dort unter dem Einfluss des Lichtes und unter Einwirkung von CO₂ die Photosynthese stattfindet, die zur Anreicherung und zum Wachstum der Algen führt. Diese Maßnahmen werden an den einzelnen Platten von ein und derselben Vorrichtung oder aber durch einen geschlossenen Kreislauf, der ausgeht vom Ausgang der einen (ersten) Vorrichtung und zum Eingang der nächsten, im Aufbau im wesentlichen gleichartigen Vorrichtung usw. hinführt, so dieser so oft durchlaufen wird, bis die gewünschte Algendichte erreicht wird.
Zur Optimierung des Ertrags werden in üblicher Weise Steuerungsvorgänge durchgeführt, die die Reaktionsparameter zur Effizienzsteigerung einstellen. Sobald eine hinreichende Algendichte erreicht wird, die gemessen über die Sauerstoffkonzentration und/oder über die Algendichte feststellbar ist, wird die Nährstofflösung abgezweigt und in einer geeigneten Station, z.B. mit Hilfe einer Zentrifuge, getrennt. Die flüssige Phase wird in den Kreislauf zurückgeleitet, hingegen die gewonnen Algen, ggf. nach einer nochmaligen Trocknung, der Weiterverwendung zugeführt werden.
Es versteht sich, dass aufgrund des Verbrauches der Nährstofflösung periodisch Nährstoffe beigefügt werden müssen.

Klarzustellen ist, dass ein Abzweigen der hinreichend angereicherten Nährstofflösung aus einem geschlossenen Produktionskreislauf und deren Zuführung zu einer Zentrifuge den Normalfall darstellen dürfte, dass dennoch bei den u. U. aus mehreren Modulen bestehende Anlagen denkbar ist, dass nach dem Durchlaufen der letzten Station (= Modul) die Nährstofflösung vollständig und als Ganzes der Trennung zugeführt werden (= nicht geschlossener Produktionskreislauf). Diese Möglichkeit gibt dann Sinn, wenn am Ende der Anlage, d.h. am letzten Modul die gewünschte Algendichte vorliegt, sodass es einer Rückführung und eines erneuten Durchlaufens der Anlage nicht mehr bedarf.

Weitere Einzelheiten, Merkmale und Vorteile der Erfindung lassen sich dem nachfolgenden Beschreibungsteil entnehmen, in dem anhand der Zeichnungen Ausführungsbeispiele der Erfindung näher erläutert werden. Es zeigen:
- Figur 1: eine erfindungsgemäße Vorrichtung in Seitenansicht
- Figur 2: eine Produktionsanlage, besehend aus mehreren miteinander verknüpften erfindungsgemäßen Vorrichtungen

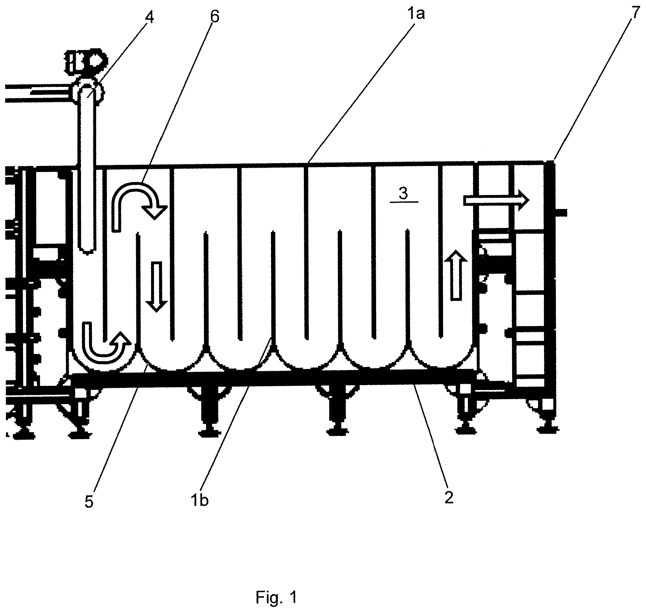

**Figur 1** zeigt in schematisch gehaltener Seitenansicht die erfindungsgemäße Vorrichtung. Sie besteht in ihrem grundsätzlichen Aufbau aus mehreren vertikal ausgerichteten und in horizontalem Abstand zueinander angeordneten Platten 1. Diese Platten sind mit ihrem Rand entweder an Oberseite des Gehäuses 1a oder am Boden 1b befestigt. Im gezeigten Ausführungsbeispiel sind die Platten 1a, 1b in alternierender Weise angeordnet, sodass jeweils einmal unterhalb der Platten 1a, zum anderen oberhalb der Platten 1b ein Abstand zum Gehäuse 2 eingehalten wird. Im Inneren des Gehäuses 2 befindet sich die Nährstofflösung 3, die zumindest soweit eingefüllt ist, dass deren Füllhöhe oberhalb der am Boden befestigten Platten 1b befindet. Bei entsprechender Beaufschlagung mit Hilfe einer Pumpe 4 bildet sich in der Nährstofflösung 3 eine Strömung aus, die mäanderförmig in der Darstellungsebene der Zeichnung verläuft. Die Strömung bewegt sich in vertikaler Richtung entlang der oberen Platte 1a nach unten, wo sie aufgrund des Abstands des unteren Rands vom Boden des Gehäuses 2 umgelenkt wird und in vertikaler Richtung zwischen der soeben beschriebenen Platte 1a und der nächsten am Boden befestigten Platte 1b nach oben strömt. Im oberen Bereich der Füllhöhe der Nährstofflösung 3 tritt die Strömung nach einer Umlenkung in die Gegenrichtung in den Zwischenraum zwischen diese soeben beschriebene Platte 1b und der nächsten oben am Gehäuse 2 befestigten Platte 1a ein um dort im wesentlichen die selben Strömungsverhältnisse auszubilden, wie sie bereits im Eingangsbereich beschrieben worden sind. Zur Verdeutlichung der Strömungsverhältnisse sind Pfeile 6 eingetragen. Im Ergebnis erhält man eine sich über die gesamte Vorrichtung erstreckende mäanderförmige Strömung.
Im unteren Bereich zum Boden des Gehäuses 2 hin sind die Platten 1b über Umlenkvorrichtungen 5 am Gehäuse 2 befestigt, die als Halbzylinderschalen geformt sind und mit ihrer Achse senkrecht zur Zeichenebene ausgerichtet sind. Hierdurch wird die Ausbildung laminarer Strömungsverhältnisse zumindest in diesem Bereich gefördert, was ein wesentlicher Beitrag zur Effizienz der gesamten Vorrichtung darstellt. Die benachbarten Umlenkvorrichtungen berühren sich randseitig, wo sie in die Platte 1b übergehen.

Mit Beendigung des Durchlaufs verlässt die nunmehr mit Mikroalgen angereicherte Nährstofflösung 3 diese Vorrichtung, um entweder bei modulmäßigem Aufbau einer nächsten, im Aufbau gleichartigen Vorrichtung oder aber einer Zentrifuge zur Trennung der Mikroalgen von der Nährstofflösung zugeführt zu werden.

Nicht eingetragen sind die an den äußersten Rändern der Platten 1 angebrachten Beleuchtungsmittel, die das Licht über die Stirnfläche in die Platte 1 einleiten und wo sich das Licht aufgrund der optisch transparenten Eigenschaften des Plattenmaterials ausbreitet. Um zu erreichen, dass das Licht über die Oberfläche der Platten wieder austritt, sind Lichtstreuende Partikel im Material der Festplatten eingebettet. Die Verteilung der Lichtstreuenden Partikel in der Platte 1 hat so zu erfolgen, dass in etwa eine konstante Dichte des über die Oberfläche der Platte 1 austretenden Lichts eintritt. Im Zusammenhang mit dem vorhandenen Kohlendioxid bewirkt das Sonnenlicht, eine Photosynthese zur Förderung des Wachstums und der Vermehrung von Phytoplankton. Zum besseren Verständnis wird die gesamte in Figur 1 gezeigte Vorrichtung mit dem Bezugszeichen 7 versehen.

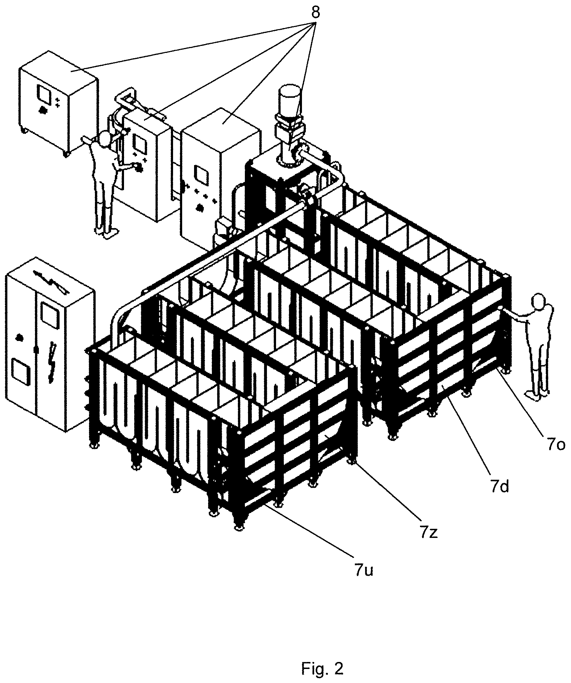

In **Figur 2** ist in schematischer Darstellung eine Produktionsanlage mit insgesamt vier Vorrichtungen 7 wiedergegeben. Dabei sind die Vorrichtungen 7 über eine Reihenschaltung miteinander verknüpft und hinsichtlich des durch die Nährstofflösung 3 gebildeten Kreislaufs miteinander in Verbindung gesetzt. Um kompakte Abmessungen der gesamten Produktionsanlage zu erreichen, sind die Vorrichtungen 7 in Zickzack-Form angeordnet.

Der Eingang zur untersten Vorrichtung 7u befindet sich etwa in der Mitte der Anlage, dort wo ein Schaltschrank symbolisch angedeutet wird. Ausgehend von dem Eingang der Vorrichtung 7u durchläuft die Nährstofflösung in Mäanderform die gesamte Vorrichtung und es werden dort entsprechend der oben beschriebenen Photosynthesereaktionen eine Vielzahl an Mikroalgen gebildet und in der Nährstofflösung 3 angereichert. Vom Ende der untersten Vorrichtung 7u ausgehend, die sich an der Stirnseite der gesamten Produktionsanlage befindet, wird die Nährstofflösung 3 in die zweite Vorrichtung 7z übergeleitet und dessen Eingang beaufschlagt.
Nach dem Durchlaufen der Vorrichtung 7z folgt eine Überleitung der Nährstofflösung 3 in die dritte Vorrichtung 7d, wobei der Übergang aufgrund der zickzackförmigen Anordnung der Vorrichtungen 7 in der Nähe des Eingangs der untersten Vorrichtung 7u stattfindet. Angelangt in der Vorrichtung 7d durchläuft die Nährstofflösung 3 die nächste und damit dritte Stufe. An deren Ende erfolgt in der vorbeschriebenen Weise die Überleitung der Nährstofflösung 3 in die oberste Vorrichtung 7o. Am Ausgang der obersten Vorrichtung 7o erfolgt eine Trennung der Nährstofflösung 3 einerseits in die Mikroalgen als das Verfahrensendprodukt und zum anderen in die Nährstofflösung, die ggf. nach einer Anreicherung zurück auf den Eingang der untersten Vorrichtung 7u gegeben wird.

Die Bauteile 8 stellen sowohl in ihrem Aufbau als auch in ihrer Funktion bekannte Gerätschaften dar und bedürfen keiner näheren Erläuterung und sie umfassen eine Zentrifuge, einen Trockner, Pumpenaggregate sowie Antriebs- und Energieversorgungseinheiten.

Die gezeigte Anordnung erlaubt auf engstem Raum die Produktion großer Mengen an Mikroalgen, was zum einen auf den Aufbau und die Wirkungsweise der einzelnen Vorrichtungen 7 sowie deren Anordnung und Verknüpfung relativ zueinander in einer im Hinblick auf den Verlauf der Nährstofflösung zickzackförmigen Anordnung zurückzuführen ist

Das Ergebnis ist eine großtechnische Produktionsanlage, durch welche eine in großen Mengen kostengünstige Herstellung von Mikroalgen möglich wird.

### Bezugszeichenliste

- 1: Platten
- 1a: an Oberseite befestigte Platte
- 1b: am Boden befestigte Platte
- 2: Gehäuse
- 3: Nährstofflösung
- 4: Pumpe
- 5: Umlenkvorrichtung
- 6: Pfeile
- 7: Vorrichtungen
- 7u: untere Vorrichtung
- 7z: 2. Vorrichtung
- 7d: 3. Vorrichtung
- 7o: oberste Vorrichtung
- 8: Bauteile

## Patentansprüche

1. Vorrichtung zur Gewinnung von Phytoplankton (Mikroalgen), bei der in einem Gehäuse (2) eine Nährstofflösung (3) und mehrere vertikal ausgerichtete und in horizontalem Abstand zueinander angeordnete Platten (1) vorhanden sind, die sich nicht bis zur gegenüberliegenden Wand erstrecken und von denen zumindest ein Teil an der Oberseite des Gehäuses (2) befestigt ist, um eine vertikal mäanderförmige Strömung auszubilden sowie die Nährstofflösung (3) über eine Pumpe (4) umgewälzt wird,
wobei an der im Bereich der Befestigung befindlichen Stirnfläche der Platten (1) Beleuchtungsmittel angebracht sind und die Platten (1) aus transparentem Festmaterial bestehen,
**dadurch gekennzeichnet, dass**
die Platten (1) alternierend entweder am Boden oder an der Oberseite des Gehäuses (2) befestigt sind und
in das Festmaterial lichtstreuende Partikel in einer geeigneten, ortsabhängigen Partikeldichte derart eingebettet sind, dass die Dichte des Lichtaustrittes über die Oberfläche der Platte (1) in etwa konstant ist.

2. Vorrichtung nach Anspruch 1, **dadurch gekennzeichnet, dass** die Beleuchtungsmittel LED und/oder OLED's sind.

3. Vorrichtung nach Anspruch 1 oder 2, **dadurch gekennzeichnet, dass** die Beleuchtungsmittel Licht aus dem Spektralbereich 430 +/- 10 nm und/oder aus dem Spektralbereich 680 +/- 10 nm emittieren.

4. Vorrichtung nach Anspruch 3, **dadurch gekennzeichnet, dass** beide Frequenzbereiche emittiert werden, wobei es bevorzugt ist, das Mischungsverhältnis von 70:30 zu Gunsten der Spektrallinien aus dem Bereich 680 +/- 10 nm beträgt.

5. Vorrichtung nach einem der vorgehenden Ansprüche, **dadurch gekennzeichnet, dass** das Material der Platte (1) Acrylglas ist.

6. Vorrichtung nach dem vorgehendem Anspruch, **dadurch gekennzeichnet, dass** das Acrylglas an der Oberfläche satiniert ist.

7. Vorrichtung nach einem der vorgehenden Ansprüche, **dadurch gekennzeichnet, dass** die Pumpe (4) eine Exzenterschneckenpumpe ist.

8. Vorrichtung nach einem der vorgehenden Ansprüche, **dadurch gekennzeichnet, dass** die Vorrichtung eine zu einer Zentrifuge führende Ausleitung ausweist.

9. Vorrichtung nach einem der vorgehenden Ansprüche, **dadurch gekennzeichnet, dass** im Bodenbereich der Umlenkung der Nährstofflösung (3) halbzylinderförmige und der Strömung angepasste Umlenkvorrichtungen (5) angeordnet sind.

10. Vorrichtung nach einem der vorgehenden Ansprüche, **dadurch gekennzeichnet, dass** zusätzlich Sonnenlicht gesammelt und in die Platte (1) eingeleitet wird.

11. Vorrichtung nach einem der vorgehenden Ansprüche, **dadurch gekennzeichnet, dass** die Vorrichtung (7) als Modul aufgebaut ist.

12. Vorrichtung nach einem der vorgehenden Ansprüche, **dadurch gekennzeichnet, dass** die Nährstofflösung (3) nach Art eines geschlossenen Kreislaufes vom Ausgang der Vorrichtung (7) wieder an den Eingang zurückgeführt wird.

13. Vorrichtung nach einem der vorgehenden Ansprüche, **dadurch gekennzeichnet, dass** die Vorrichtung (7) Messfühler zur Erfassung der Temperatur und/oder elektrischen Leitfähigkeit und/oder des pH-Wertes und/oder der Sauerstoffkonzentration und/oder der optischen Dichte aufweist.

14. Vorrichtung nach einem der vorhergehenden Ansprüche, **dadurch gekennzeichnet, dass** Leitungen zur Einspeisung von CO₂ und/oder Leitungen zum Absaugen von O₂ angeordnet sind.

## Claims

1. Device for the collection of phytoplankton (microalgae), in which, in a housing (2), a nutrient solution (3) and a plurality of vertically oriented plates (1) are disposed at a horizontal spacing to one another, which do not extend as far as the opposite wall and of which at least a portion are fastened on the top side of the housing (2) to form a vertically meandering stream, as well as in which the nutrient solution (3) is recirculated via a pump (4),
wherein, at the end faces of the plates (1), in the region of the fastening, illumination means are mounted and the plates (1) consist of transparent solid material, **characterised in that** the plates (1) are fastened alternately either on the base or on the top side of the housing (2), and, in the solid material, light-scattering particles are embedded, with a suitable location-dependent particle density, such that the density of the light emergence is approximately constant over the surface of the plate (1).

2. Device according to claim 1, **characterised in that** the illumination means are LEDs and/or OLEDs.

3. Device according to claim 1 or 2, **characterised in that** the illumination means emit light in the spectral range 430 +/-10 nm and/or in the spectral range 680 +/-10 nm.

4. Device according to claim 3, **characterised in that** both frequency ranges are emitted, it being preferred that the mixing ratio is 70:30 in favour of the spectral lines from the range 680 +/-10 nm.

5. Device according to one of the preceding claims, **characterised in that** the material of the plate (1) is acrylic glass.

6. Device according to the preceding claim, **characterised in that** the acrylic glass is satinized on the surface.

7. Device according to one of the preceding claims, **characterised in, that** the pump(4) is an eccentric screw pump..

8. Device according to one of the preceding claims, **characterised in that** the device has a discharge line leading to a centrifuge.

9. Device according to one of the preceding claims, **characterised in that,** in the base region of the deflection of the nutrient solution (3) semicylindrical deflection devices (5), which are adapted to the flow, are disposed.

10. Device according to one of the preceding claims, **characterised in that** sunlight is additionally collected and guided into the plate (1).

11. Device according to one of the preceding claims, **characterised in that** the device (7) is constructed as a module.

12. Device according to one of the preceding claims, **characterised in that** the nutrient solution (3) is fed back from the outlet of the device (7) to the inlet in the manner of a closed circuit.

13. Device according to one of the preceding claims, **characterised in that** the device (7) comprises a measurement sensor for registering the temperature and/or electrical conductivity and/or the pH and/or the oxygen concentration and/or the optical density.

14. Device according to one of the preceding claims, **characterised in that** lines for feeding CO₂ and/or lines for suction of O₂ are disposed.

## Revendications

1. Dispositif destiné à récolter du phytoplancton (microalgues), dans lequel un boîtier (2) contient une solution nutritive (3) et plusieurs plaques (1) orientées à la verticale et disposées horizontalement à une certaine distance les unes des autres, qui ne s'étendent pas jusqu'au mur situé en face et dont au moins une partie sont fixées sur la partie supérieure du boîtier (2) afin de former un courant vertical en forme de méandre et pour que la solution nutritive (3) soit mise en circulation par une pompe (4),
sachant que des moyens d'éclairage sont disposés sur la face frontale des plaques (1) se trouvant dans la zone de la fixation et que les plaques (1) consistent en matériau fixe transparent,
**caractérisé par le fait que**
les plaques (1) sont fixées de façon alternée au sol ou sur la face supérieure du boîtier (2) et que des particules diffusant la lumière sont intégrées dans le matériau fixe dans une densité de particules dépendant du lieu adaptée de façon à ce que la densité de la sortie de la lumière sur la surface de la plaque (1) soit à peu près constante.

2. Dispositif selon la revendication 1, **caractérisé par le fait que** les moyens d'éclairage sont des LED et/u des OLED.

3. Dispositif selon la revendication 1 ou 2, **caractérisé par le fait que** les moyens d'éclairage émettent de la lumière à partir de la plage spectrale 430 +/- 10 nm ou à partir de la plage spectrale 680 +/-10 nm.

4. Dispositif selon la revendication 3, **caractérisé par le fait que** deux plages de fréquence sont émises, sachant que le rapport de mélange de 70:30 est préféré en faveur des lignes spectrales issues du domaine 680 +/- 10 nm.

5. Dispositif selon une des revendications précédentes, **caractérisé par le fait que** le matériau de la plaque (1) est du verre acrylique.

6. Dispositif selon la revendication précédente, **caractérisé par le fait que** le verre acrylique est satiné à sa surface.

7. Dispositif selon une des revendications précédentes, **caractérisée par le fait que** la pompe (4) est une pompe à vis excentrique.

8. Dispositif selon une des revendications précédentes, **caractérisée par le fait que** le dispositif présente une conduite de sortie menant à une centrifugeuse.

9. Dispositif selon une des revendications précédentes, **caractérisé par le fait que** des dispositifs de renvoi (5) de forme semi-cylindrique et adaptés au courant sont disposés dans la zone du fond du renvoi de la solution nutritive (3).

10. Dispositif selon une des revendications précédentes, **caractérisé par le fait que** de la lumière solaire supplémentaire est collectée et amenée à la plaque (1).

11. Dispositif selon une des revendications précédentes, **caractérisé par le fait que** le dispositif (7) est réalisé sous la forme d'un module.

12. Dispositif selon une des revendications précédentes, **caractérisé par le fait que** la solution nutritive (3) est ramenée à l'entrée sous la forme d'un circuit fermé depuis la sortie du dispositif (7).

13. Dispositif selon une des revendications précédentes, **caractérisé par le fait que** le dispositif (7) présente des sondes de mesure pour enregistrer la température et/ou la conductivité électrique et/ou le taux de pH et/ou la concentration en oxygène et/ou la densité optique.

14. Dispositif selon une des revendications précédentes, **caractérisé par le fait que** des conduites destinées à alimenter en CO² et des conduites destinées à aspirer l'O² sont disposées.
